# EUROPEAN PATENT SPECIFICATION

(11) **EP 1 713 520 B1**
(45) Date of publication and mention of the grant of the patent: **23.12.2009**
(21) Application number: 05708343.8
(22) Date of filing: 11.02.2005
(51) Int. Cl.: A61L 12/10, C11D 3/02, C11D 3/24

(54) **CLEANING COMPOSITION**
REINIGUNGSZUSAMMENSETZUNG
COMPOSITION DE NETTOYAGE

(30) Priority: 11.02.2004 GB 0402947
(43) Date of publication of application: 25.10.2006
(73) Proprietor: Amity Limited, Barnsley South Yorkshire S75 3SP (GB)
(72) Inventor: MALYSZEWICZ, Christopher, Bugbrooke, Northamptonshire NN7 3QT (GB)
(74) Representative: Franks & Co (South) Limited
(86) International application number: PCT/GB2005/000525
(87) International publication number: WO 2005/089820

(56) References cited:
- WO-A-03/073849

## Description

The present invention relates to a liquid cleaning composition with both an anti-viral activity and an anti-bacterial action. More particularly but not exclusively, it relates to such a composition for cleansing hard surfaces, although it may also relate to such compositions for cleaning wares, textiles or medical implements. It may also relate to such a composition incorporated into a paint or other surface coating composition.

In the medical field, concern is growing about both patients and staff picking up bacterial and/or viral infections in hospitals or the like. It is believed that a significant vector for such infection is imperfectly disinfected surfaces harbouring bacteria and/or viruses that are or have become resistant to existing surface cleaning agents. For example, it is suspected that the spread of the recent SARS (Severe Acute Respiratory Syndrome) outbreak was connected to the SARS virus having a significant resistance to existing cleaning agents and topical disinfectants. Thus, viruses from an infected patient may survive existing cleaning compositions and regimes, and be picked up by other patients and medical staff, spreading the infection further.

Other so-called "superbugs", such as the MRSA bacterium, are believed to be capable of surviving existing surface cleaning materials and regimes, and lingering to infect further victims. Since such bacteria are treatable by very few antibiotics, once infection has taken place, it is most important that they be thoroughly eradicated before cross-infection can occur.

Existing cleaning agents often rely on cationic surfactants, such as quaternary ammonium salts, which act as both surface cleaning agents and bactericides. However, while known quaternary ammonium salts are sufficiently active to deal with almost all bacteria, etc, that might cause food-poisoning in a domestic or catering environment, they are not viewed as being sufficiently powerful to eliminate, reliably, the more resistant and/or more dangerous pathogens causing concern in hospitals and the like.

While neat alcohols, such as *iso*-propanol, are used for topical disinfections around wounds or to prepare the site of an injection, their use is not practical for widespread application to disinfect hard surfaces and so forth.

Another class of biocidal reagents which are lethal to a wide range of organisms is the halogens. However, bleach and other chlorine sources are too crude for many applications, for example being incompatible with some surfaces to be cleaned, potentially causing bums to the unprotected skin of a person cleaning with them, and emitting toxic fumes. Lower halogens, such as bromine, are a little less hazardous, but may be intensely coloured at useful concentrations, leading to staining problems.

While cleaning of surfaces is a current focus of interest, removal of harmful microorganisms from textile products (e.g. sheets, curtains and bedclothing) would also be most beneficial. Similarly, a warewashing composition capable of removing such contamination from crockery and cutlery used by infected patients would be highly desirable; such a composition might also be of use in cleaning medical equipment and implements.

Furthermore, it would be beneficial to incorporate such anti-viral and anti-bacterial properties into a paint, varnish or other surface coating composition, to give hard surfaces in-built protection.

International Patent Application No. WO 03/073849 A1 discloses a range of diester dicarboxylate antimicrobial compositions. Amongst the components disclosed are interhalides, such as bromine chloride, iodine monobromide and iodine dibromide; cosolvents such as alcohols; and cationic surfactants including ethoxylated and/or propoxylated alkyl amines, diamines, or triamines.

It is hence an object of the present invention to provide a liquid surface cleaning composition obviating the above disadvantages while having an anti-viral and anti-bacterial activity sufficient to handle surface contamination by pathogens of the types described. It is also an object of the present invention to provide a textile, ware or medical instrument cleaning composition or a surface coating composition having such properties.

According to a first aspect of the present invention, there is provided an aqueous surface cleaning and disinfecting composition comprising bromine, a bromine complex and/or a bromine-releasing compound, a long-chain alkyl triamine compound having the general formula R-N((CH₂)ₘNH₂)(CH₂)ₙNH₂, where R is a linear or branched alkyl chain comprising at least eight carbon atoms and each of m and n equals 2, 3 or 4, and one or more aliphatic alcohols.

Preferably, the composition comprises at least one polyglycol ether, optionally a poly(ethylene glycol) ether or a poly(propylene glycol) ether.

The long-chain alkyl triamine compound may comprise a compound having the general formula R-N((CH₂)ₘNH₂)₂, i.e. m equals n.

Each of m and n may equal 3.

R may be a linear or branched alkyl chain comprising between ten and sixteen carbon atoms.

The composition may comprise a mixture of such long-chain alkyl triamine compounds having different chain lengths.

Preferably, said one or more aliphatic alcohols each comprise between one and four carbon atoms.

Advantageously, said one or more aliphatic alcohols are each primary alcohols.

The composition may comprise two different aliphatic alcohols, optionally ethanol and *n-*propanol.

Preferably the composition comprises between 5% and 25% by volume aliphatic alcohols.

The composition may comprise between 10% and 20% by volume aliphatic alcohols.

The composition may comprise between 5% and 15% by volume ethanol, optionally between 10% and 12% ethanol.

The composition may comprise between 1% and 10% by volume *n*-propanol, optionally between 4% and 6% *n*-propanol.

Preferably, the composition comprises up to 0.1% by weight bromine.

Advantageously, the composition comprises between 0.01 % and 0.05% by weight bromine.

Optionally, the composition comprises between 0.01 % and 0.02% by weight bromine.

Preferably, the composition comprises between 10% and 30% by volume of the long-chain alkyl polyamine compound or compounds.

Advantageously, the composition comprises between 15% and 25% by volume of the long-chain alkyl polyamine compound or compounds, optionally 20% ± 2%.

The composition may comprise between 5% and 15% by volume of the at least one polyglycol ether.

According to a second aspect of the present invention, there is provided a composition adapted for cleaning textiles and the like comprising bromine, a bromine complex and/or a bromine-releasing compound, a long-chain alkyl polyamine compound, and one or more aliphatic alcohols.

Preferably, said textile cleaning composition comprises a composition as described in the first aspect above.

According to a third aspect of the present invention, there is provided a ware-washing composition comprising bromine, a bromine complex and/or a bromine-releasing compound, a long-chain alkyl polyamine compound, and one or more aliphatic alcohols.

Preferably, said ware-washing composition comprises a composition as described in the first aspect above.

Advantageously, said ware-washing composition is adapted for use in cleaning medical and/or surgical equipment and implements.

According to a fourth aspect of the present invention, there is provided a surface coating composition comprising bromine, a bromine complex and/or a bromine-releasing compound, a long-chain alkyl polyamine compound, and one or more aliphatic alcohols.

Preferably, said surface coating composition comprises an aqueous surface coating composition.

Advantageously, said surface coating composition comprises a composition as described in the first aspect above.

Embodiments of the present invention will now be more particularly described by way of example.

An aqueous surface cleaning composition was prepared, comprising:

| | |
|---|---|
| NTA 89% powder | 1.36kg |
| Ethanol | 11.39 litres |
| *n*-Propanol | 5.0 litres |
| Topanol O FG | 0.55 kg |
| Synperonic A9 (90%) | 6.95 litres |
| Synperonic A7 | 3.42 litres |
| Sandoteric ABD | 4.45 litres |
| Triameen Y12D-30 | 19.865 litres |
| Deionised water | 47.0 litres |
| Bromine GPR | 0.015kg |

The composition appeared as a clear pale yellow liquid with a pH of approximately 8 and a mildly alcoholic odour.

NTA is the trisodium salt of nitrilotriacetic acid, and acts as a buffering agent. Topanol O FG is butylated hydroxytoluene (food grade), an antioxidant, obtainable from Chance and Hunt Ltd. (Topanol is a registered trade mark of ICI plc). Synperonic A7 and Synperonic A9 are polyethoxylate ethers, produced by the Uniqema business of ICI plc and available via Albion Chemicals Ltd. (Synperonic is a registered trade mark of ICI Chemicals and Polymers Ltd). Sandoteric ABD is a complex blend of amphoteric surfactants acting as a detergent and having a degree of bactericidal activity, sold by Clariant. (Sandoteric is a registered trade mark of Novartis SA). Triameen Y12D-30 is a long-chain alkyl triamine of the general formula R'-N(C₃H₆NH₂)₂, where R' is a "tallow alkyl" - a naturally-derived mixture of alkyl chains having a range of different lengths. It is sold by Akzo Nobel.

The triamine is believed to form a cationic species at a pH such as is produced by buffering with NTA. The polyethoxylate ethers not only perform their conventional role as wetting agents, but also form relatively stable complexes with the bromine. The composition thus has several active components with antiviral and/or anti-bacterial properties - the two alcohols, the cationic triamine species and the complexed bromine. These have a synergistic effect, particularly against the more resistant viruses and bacteria, beyond what would be expected from the individual active components. It is believed that the phospholipid membranes that form the capsid of a virus on the outer cell wall of a bacterium are attacked, leading to lysis or rupture, respectively. The viral RNA or bacterial DNA thus released is attacked and destroyed or complexed and inactivated. For example, viral RNA is susceptible to cleavage via attack by alcohols on the carboxyl groups of lysine or arginine, while the cationic triamine may bind to critical portions of the helix of bacterial DNA, interfering with its replication. The particular combination of active components used allows attack on both hydrophilic and hydrophobic lipid membranes. As a result, there appears to be no class of bacteria or viruses upon which the composition cannot act.

A small proportion of viruses or bacteria may survive without being lysed or ruptured, but such survivors appear to be so damaged that they are unable to penetrate a cell wall to infect the cell. This inactivation is believed to last for two weeks or more, significantly longer than can be achieved with existing disinfectant cleaners.

The composition also acts as a powerful detergent, breaking up and dispersing surface soiling, washing off undestroyed bacteria/viruses and removing the debris of those destroyed. In a formulation as described, the expected staining problems from the bromine are found not to be an issue.

The composition will also act to dislodge fungal, mould and yeast contamination, and has a degree of biocidal activity against them, although the walls of their spore cells are considerably tougher than those of bacteria.

The two Synperonic polyethoxylate ethers differ only in the average number of ethylene oxide units per molecule that they contain: A7 has seven units, while A9 has nine. They both have the same alkyl chain, derived from a branched fully saturated primary alcohol having thirteen to fifteen carbon atoms. The hydrophilic-lipophilic balance (HLB) value for Synperonic A7 is 12.2, so it is just water soluble/dispersible, while the HLB value of Synperonic A9 is 13.0, making it appreciably more water soluble. The Sandoteric ABD amphoteric detergent blend is one of several broadly equivalent products available on the market. It is hence possible to vary the above formulation slightly depending on the availability and cost of raw materials, while maintaining its effectiveness.

Even initial results have indicated that compositions as described above can be expected to show wide ranging effectiveness against both bacteria and viruses, including the particularly resistant and/or dangerous examples currently causing concern.

In a first series of tests, the above composition was tested for its effectiveness against a range of well-known potentially harmful microorganisms, comprising Staphylococcus aureus (strain NCTC 10788), Pseudomonas aeruginosa (strain NCTC 6749), Escherichia coli (strain NCTC 10418), Enterococcus hirae (strain NCTC 12367) and Methicillin resistant Staphylococcus aureus (strain NCTC 12493), generally known as MRSA. The test was carried out according to the method laid out in British Standard EN1276 "Chemical disinfectants and antiseptics - Quantitative suspension test for the evaluation of bactericidal activity of chemical disinfectants and antiseptics used in food, industrial, domestic and institutional areas (Phase 2, step 1): Tests for disinfectants for medical establishments not yet ratified," with some minor alterations to make the test more demanding.

In summary, the test comprises mixing 1 ml of a test bacteria preparation with 1 ml of a model "soil" solution. For a test simulating "clean" conditions, the "soil" comprises 0.03% bovine albumin, while for a test simulating "dirty" conditions, the "soil" comprises 0.3% bovine albumin. To this mixture is added 8ml of the composition under test, diluted to an "in-use" concentration (in these tests, 1 part composition to 9 parts sterile hard water). In the standard EN 1276 test, this is left for a five minute contact time. In the present example, contact times of 1, 2 and 5 minutes were used, to increase the severity of the test. After the selected contact time, 1ml of the mixture is removed and mixed with 9ml of a recovery/neutraliser solution, in order to halt the action of the disinfectant composition. The standard recovery/neutraliser solution from EN1276 is 30 grams per litre Tween 80, 3 grams per litre lecithin and 20ml per litre sodium di-octyl sulphosuccinate (Tween is a registered trade mark owned by ICI Americas Inc). This proved suitable for the gram-negative test organisms, (P. aeruginosa and E. coli), but not for the other, gram-positive test organisms, unless a higher than standard initial bacterial count was used. After neutralisation, the sample is plated to detect and assay the surviving test bacteria. Bacterial counts are expressed as a log₁₀ of the number counted. In the standard form of EN1276, an initial (challenge) level of around 7 is used. To pass the test, the count should be reduced by at least 5 - i.e. a drop in bacterial concentration of over five orders of magnitude or a better than 99.999% bacterial kill rate. In the variant of EN1276 used for E.hirae, S.aureus and MRSA, the challenge level was just over 9. Again, a reduction of at least 5 was required to pass. Tests are carried out in duplicate.

The test results for the composition exemplified were:

| **Log₁₀ reduction** | | | | | | | |
|---|---|---|---|---|---|---|---|
| **Test organism** | **Log₁₀ initial Count(challenge)** | **Clean conditions (0.03% albumin)** | | | **Dirty Conditions (0.3% albumin)** | | |
| | | 1 min | 2 mins | 5 mins | 1 min | 2 mins | 5 mins |
| ***Ps. aeruginosa*** | 7.30 | >6.30 | >6.30 | >6.30 | >630 | >6.30 | >6.30 |
| ***Esch. coli*** | 7.00 | >6.00 | >6.00 | >6.00 | >6.00 | >6.00 | >6.00 |
| ***Ent. hirae*** | 9.60 | >6.60 | >6.60 | >6.60 | >6.60 | >6.60 | >6.60 |
| ***Staph. aureus*** | 9.20 | >6.20 | >6.20 | >6.20 | >6.20 | >6.20 | >6.20 |
| ***Methicillin resistant Staph. aureus*** | 9.04 | >6.04 | >6.04 | >6.04 | >6.04 | >6.04 | >6.04 |

Thus, against each organism tested, with 1, 2 or 5 minute contact times, the composition of the present invention achieves a reduction of more than six orders of magnitude, easily passing the test criteria.

A second series was carried out against MRSA alone (multi-loop Staphylococcus aureus, MRSA strain ATCC 43300), again using the method laid out in British Standard EN1276 (Phase 2 step 1) with modifications to increase the stringency.

The composition was again used diluted by 1 part composition to 9 parts sterile hard water. In place of the standard five minute contact time, contact times of 30, 60 and 90 seconds were used. 1ml of the test bacteria preparation was mixed with 1ml of "soil" (again, either "clean" 0.03% bovine albumin or "dirty" 0.3% bovine albumin). 19ml of the composition at its in-use concentration was added to this, and after the selected contact time, 1ml of the mixture was removed and diluted/neutralised with 19ml of the above recovery/neutraliser solution, before being plated as above. The initial MRSA challenge level was again around 9, with a target of a reduction of at least 5.

The test results were:

| **Log₁₀ Initial count (challenge)** | **Clean conditions** | | | **Dirty Conditions** | | |
|---|---|---|---|---|---|---|
| | 30 secs | 60 secs | 90 secs | 30 secs | 60 secs | 90 secs |
| **9.08** | >4.90 | >6.50 | >7.20 | >4.60 | >5.90 | >6.80 |

Thus, the composition of the present invention easily achieved the required reduction of five orders of magnitude with a contact time of 60 or 90 seconds. With a contact time of only 30 seconds (as against five minutes for the standard EN1276 test), the composition still almost reached the target reduction.

A third series of tests was then carried out, using the composition diluted even further (1 part composition to 20 parts sterile hard water) to increase the severity of the test. MRSA (strain ATCC 43300) was again employed, starting with a challenge count of around 9. Compared to the second series of tests, the method was identical, except that 1ml of the test bacteria preparation was mixed with 1ml of "clean" or "dirty" soil and 9ml of the composition at its in-use concentration; other steps were unchanged.

The test results were:

| **Log₁₀ Initial Count (challenge)** | **Clean conditions** | | | **Dirty Conditions** | | |
|---|---|---|---|---|---|---|
| | 30 secs | 60 secs | 90 secs | 30 secs | 60 secs | 90 secs |
| **9.08** | >3.95 | >5.55 | >6.95 | >3.25 | >5.00 | >6.25 |

Thus, the composition of the present invention achieved the required reduction in bacterial concentration of five orders of magnitude with a contact time of 60 or 90 seconds. However, at this concentration, a contact time of well over 30 seconds was required to pass the test (again, compared to five minutes available in the standard EN1276 test).

In a fourth series of tests, the composition was tested diluted by 1 part composition to 99 parts sterile hard water. MRSA (strain ATCC 43300) was again used with a challenge count of around 9. Compared to the second and third series, all steps were unchanged, except that 99ml of the composition at its in-use concentration was employed, and the recovery/neutralisation step was performed with only 9ml of the above recovery/neutralisation solution.

The test results were:

| **Log₁₀ Initial Count (challenge)** | **Clean conditions** | | | **Dirty Conditions** | | |
|---|---|---|---|---|---|---|
| | 30 secs | 60 secs | 90 secs | 30 secs | 60 secs | 90 secs |
| **9.00** | >2.88 | >5.1 0 | >6.11 | >2.55 | >4.88 | >5.85 |

Thus, under "clean" soiling conditions, the composition achieved the required reduction, even at a hundredfold dilution, with a contact time of 60 or 90 seconds. Under "dirty" soiling conditions, the composition at a hundredfold dilution narrowly missed the target reduction with a contact time of 60 seconds, but passed it easily with a contact time of 90 seconds. In either case, a contact time of 30 seconds gave a significant count reduction, but well short of the target.

In all, even at a hundredfold dilution, and in "dirty" conditions, the present composition is able to pass the target reduction of EN 1276 with little more than a fifth of the contact time allowed in the standard test.

A fifth series of tests was carried out to examine the composition's efficacy against viral contamination. The test method used a challenge of poliovirus as a surrogate for Norwalk virus or small round structural viruses (SRSVs). These are RNA viruses like poliovirus, and are potential contaminants of hard surfaces in a hospital environment, but are impossible to grow in tissue culture to produce a challenge sample.

The test involved a quantitative assay of viral RNA remaining after treatment with the composition. Viral inactivation usually occurs by damaging the outer viral lipoprotein capsid, which is an essential part of the virus structure for its infection mechanism. It is a reasonable assumption that inactivation by lipoprotein degradation will occur before degradation of the virus' RNA internal payload proceeds, and so an assay on remaining RNA is a good surrogate for virucidal activity, with a substantial margin of safety built in.

The composition was tested by exposure to poliovirus vaccine for 5, 30 and 60 minutes at room temperature (18-21°C), compared with a 60 minute control of water to allow for viral degradation not due to the action of the composition.

The composition was diluted to an in-use composition (1 part composition to 9 parts water). Samples of 900µl aliquots of poliovirus vaccine at three different concentrations (1 x 10⁵ TCID₅₀/ml; 1 x 10⁴ TCID₅₀/ml; 1 x 10³ TCID₅₀/ml; "TCID₅₀/ml" means "tissue culture infective doses per millilitre", a standard unit of virus concentration). The controls comprised 900µl of water plus 100µl aliquots of vaccine at each of the above concentrations.

After 5, 30 and 60 minutes, a 200µl aliquot was removed from each test sample and subjected to a routine extraction protocol to isolate remaining viral RNA. This protocol comprised adding the above 200µl aliquot to 200µl of Binding buffer plus poly (A) and 50µl proteinase K. The mixture was vortexed and briefly centrifuged before being incubated at 72°C for one minute. After a further brief centrifugation, 100µl of 100% isopropanol was added, and the mixed sample was applied to a high pure filter tube and centrifuged at 8000*g* for one minute. The flow through material was discarded and the filter was treated with 250µl standard inhibitor removal buffer and 250µl wash buffer, with a one minute centrifugation at 8000*g* between each step. Lastly, 200µl prewarmed elution buffer was applied to the column followed by a final centrifugation at 8000*g* for one minute.

The result extract was tested for the presence of viral RNA by using a very sensitive quantitative PCR assay which amplifies a section of viral genome approximately 180bp (base pairs) in length.

The results of this test are expressed as the percentage reduction in the assayed TCID₅₀/ml value:

| | **% Reduction** | | |
|---|---|---|---|
| **Exposure** | **1 x 10⁵ TCID₅₀/ml** | **1 x 10⁴ TCID₅₀/ml** | **1 x 10³ TCID₅₀/ml** |
| 5 Minutes | 61.86% | 59.36% | 58.24% |
| 30 Minutes | 62.70% | 64.81% | 69.82% |
| 60 Minutes | 63.29% | 64.77% | ND* |
| | | | |
| 60 minutes control | 1 x 10⁵ | 9 x 10³ | 1 x 10³ |

ND indicates Not Detected; the remaining virus concentration was below the limits of the assay method (believed to be 0.4 TCID₅₀/ml).

These results indicate a significant degradation of the poliovirus RNA, even after 5 minutes exposure to the composition of the present invention. It is hence reasonable to deduce that the viral capsid had been degraded by this time, and that the composition, diluted 1:9 with water, would be an effective virucidal agent against poliovirus, Norwalk agent and SRSVs.

A sixth series of tests examined the composition's effectiveness against tubercular contamination. Since there is as yet no finally agreed British Standard or EN test for tuberculocidal activity, a method was used as described in Griffiths et al, Journal of Hospital Infection (1998) 38, 183-192.

The composition was diluted, by 1 part composition to 4 parts sterile hard water. In place of the pathogen Mycobacterium tuberculosis, Mycobacterium terrae (strain NCTC 10856/ATCC 15755) was used as a surrogate test organism. M. terrae is now recognised as a useful model for testing tuberculocidal activity (CEN TC216).

A sample of the type strain of M. terrae was obtained freeze dried from the National Collection of Type Cultures (NCTC), Central Public Health Laboratory, Colindale, London. A small aliquot of 7H0 broth was added to the glass vial containing the sample to rehydrate the organisms. 100µl of the resulting suspension was spread on Middlebrook 7H11 agar with OADC supplement (supplied by Becton Dickinson Ltd) and incubated at 37°C for 14 days.

One colony of the M. terrae test organism was taken from the agar after this incubation and was inoculated into 100ml 7H9 broth and incubated at 37°C for a further 21 days. The suspension was treated ultrasonically for 10 minutes every second day and inverted several times, to minimise clumping. Ten percent glycerol was added as a preservative and to maintain a homogeneous suspension. Aliquots of 1ml of the final suspension were decanted into 1.5ml centrifuge tubes and stored at -70°C until required.

For a test, one of the above aliquots was thawed to room temperature, centrifuged, washed twice and spread on a 7H11 plate for confluent growth, using a sterile swab. One loopful was also spread on a 7H11 plate for single colonies to confirm the purity of the suspension. After 14 days incubation at 37°C, the growth was harvested from the plate and mixed for five minutes with glass beads, moistened with sterile distilled water, in a sterile bottle. 10ml of sterile distilled water was added to the beads, shaken and the mixture left to settle for ten minutes. The supernatant was then transferred to a second sterile bottle and left to settle under gravity for two hours. The supernatant from this step was used as the test suspension of Mycobacterium terrae in subsequent tests.

A 100µl aliquot of the M. terrae test suspension was added to 900µl of the diluted composition in a microcentrifuge tube. After a contact time of 1, 3, 5, 10 or 20 minutes, a 10µl sample was extracted and added to 990µl of a neutralisation/recovery medium, comprising a mixture of lecithin, Tween, saponin, sodium thiosulphate and histidine, which is becoming standard in mycobacterial assays.

The neutralised sample was then serially diluted to 10⁻³ of its original concentration in Ringers solution. 100µl of the undiluted neutralised sample and each of the subsequent dilutions was spread on to 7H11 agar, in duplicate, using sterile spreaders. The agar plates were incubated at 37°C and checked for growth after two weeks incubation, and every week thereafter for up to four weeks.

As a model for "dirty" conditions, the test was repeated in the presence of 1% horse serum.

As for the EN1276 test above, a reduction in bacterial count of 5 or more (i.e. 99.999% or better kill rate) was used as an indication of high/intermediate level disinfection and tuberculocidal activity.

The results were:

| | **Freshly Prepared** | |
|---|---|---|
| **Contact time** | **Clean conditions** | **Dirty conditions** |
| 0 mins (challenge level) | 8.45 | 8.45 |
| 1 min (reduction by) | 2.03 | 1.77 |
| 2 mins | 2.22 | 2.23 |
| 5 mins | 3.39 | 3.32 |
| 10 mins | 5.27 | 5.05 |
| 20 mins | >5.45 | >5.45 |
| 30 mins | >5.45 | >5.45 |

The target reduction in tubercular bacterial count was thus achieved after a 10 minute contact time. This is believed to be an excellent result.

A seventh series of tests was carried out to test the effectiveness of the composition against the micro-fungal organisms Aspergillus niger and Candida albicans. This test was carried out in accordance with British Standard EN1276, as above.

For A. niger, a test concentration of 1.28 x 10⁹ cfu/ml was used (cfu = colony forming unit, a standard unit in microbiology), while for C. albicans, a test concentration of 1.30 x 10⁸ cfu/ml was used. These samples were contacted with the composition for 1, 5 or 15 minutes.
In each case, a reduction of much better than 99.999% in microorganism level, assessed in cfus, was achieved.

The composition is clearly highly effective against microfungal organisms.

Overall, these tests demonstrate a high degree of effectiveness against microfungal organisms, viruses and bacteria, including bacteria of significant concern such as MRSA.

The aqueous surface cleaning composition described above is also envisaged to be effective incorporated into a textile cleaning composition, such as a washing powder or a liquid detergent, for use in a washing machine or the like to clean such materials as curtains, sheets, patients' pyjamas, nightdresses and the like, medical staff uniforms and so forth.

A similar composition is showing promise in cleaning wares - i.e. cutlery, crockery, glassware, cooking or serving vessels and the like - which may become contaminated with micro-organisms, for example from infected patients. It is also envisaged that this composition will be of use in cleaning harmful micro-organisms from medical instruments and the like. The composition for use in ware-washing or medical instrument cleaning contains substantially higher levels of the alkyl triamine (Triameen) than the surface cleaning composition described above. Whereas a 30% active content preparation of the triamine may be used in producing the surface cleaning composition, a 100% active content preparation appears preferable for warewashing/medical instrument cleaning.

Another similar composition may be incorporated into a paint, varnish, or other surface coating composition (preferably a water-based surface coating composition), which may be applied to a hard surface, such as a wall or a floor, at risk from viral and bacterial contamination. Thus, the hard surface is provided with innate anti-bacterial and anti-viral protection, effective between (or even instead of) washes with the above surface cleaning composition. To formulate an effective water-based paint or the like, the wetting agents (Synperonic A7/A9) and the surfactant/detergent (Sandoteric ABD) are omitted from the above surface cleaning composition formulation, since corresponding materials are already present in conventional aqueous surface coating formulations.

## Claims

1. An aqueous surface cleaning and disinfecting composition **characterised in that** it comprises at least one long-chain alkyl polyamine compound having the general formula R-N((CH₂)ₘNH₂)(CH₂)ₙNH₂ where R is a linear or branched alkyl chain comprising at least eight carbon atoms and each of m and n equals 2, 3 or 4, one or more aliphatic alcohols, and bromine, a bromine complex and/or a bromine-releasing compound.

2. A composition as claimed in claim 1, **characterised in that** it further comprises at least one polyglycol ether, optionally a poly(ethylene glycol) ether or a poly(propylene glycol) ether.

3. A composition as claimed in either claim 1 or claim 2, **characterised in that** the or each long-chain alkyl triamine compound comprises a compound having the general formula R-N((CH₂)ₘNH₂)₂.

4. A composition as claimed in any one of the preceding claims, **characterised in that** each of m and n equals 3.

5. A composition as claimed in any one of the preceding claims, **characterised in that** the or each long-chain alkyl triamine compound has a linear or branched alkyl chain comprising between ten and sixteen carbon atoms.

6. A composition as claimed in any one of the receding claims, **characterised in that** said one or more aliphatic alcohols each comprise between one and four carbon atoms.

7. A composition as claimed in any one of the preceding claims, **characterised in that** the or each of said one or more aliphatic alcohols is a primary alcohol.

8. A composition as claimed in any one of the preceding claims, **characterised in that** it comprises two different aliphatic alcohols, optionally ethanol and *n*-propanol.

9. A composition as claimed in any one of the preceding claims, **characterised in that** it comprises between 5% and 25% by volume aliphatic alcohols, optionally between 10% and 20% aliphatic alcohols.

10. A composition as claimed in any one of the preceding claims, **characterised in that** it comprises up to 0.1% by weight bromine.

11. A composition as claimed in any one of the preceding clams, **characterised in that** it comprises between 0.01 % and 0.05% by weight bromine.

12. A composition as claimed in any one of the preceding claims, **characterised in that** it comprises between 10% and 30% by volume of the long-chain alkyl thiamine compound or compounds, optionally between 15% and 25% by volume thereof.

13. A composition for cleaning textiles and the like, **characterised in that** it comprises at least one long-chain alkyl triamine compound having the general formula R-N((CH₂)ₘNH₂)(CH₂)ₙNH₂ where R is a linear or branched alkyl chain comprising at least eight carbon atoms and each of m and n equals 2, 3 or 4, one or more aliphatic alcohols, and bromine, a bromine complex and/or a bromine-releasing compound.

14. A warewashing composition **characterised in that** it comprises at least one long-chain alkyl triamine compound having the general formula R-N((CH₂)ₘNH₂)(CH₂)ₙNH₂ where R is a linear or branched alkyl chain comprising at least eight carbon atoms and each of m and n equals 2, 3 or 4, one or more aliphatic alcohols, and bromine, a bromine complex and/or a bromine-releasing compound.

15. A surface coating composition **characterised in that** it comprises at least one long-chain alkyl triamine compound having the general formula R-N((CH₂)ₘNH₂)(CH₂)ₙNH₂ where R is a linear or branched alkyl chain comprising at least eight carbon atoms and each of m and n equals 2, 3 or 4, one or more aliphatic alcohols, and bromine, a bromine complex and/or a bromine-releasing compound.

## Patentansprüche

1. Wässrige Oberflächenreinigungs- und Desinfektionszusammensetzung, **dadurch gekennzeichnet, dass** sie mindestens Folgendes umfasst: eine langkettige Alkylpolyaminverbindung, die folgende allgemeine Formel aufweist: R-N((CH₂)ₘNH₂)(CH₂)ₙNH₂, wobei R eine lineare oder verzweigte Alkylkette ist, die Folgendes umfasst: mindestens acht Kohlenstoffatome, wobei m und n gleich 2, 3 oder 4 ist, einen oder mehrere aliphatische Alkohole und Brom, einen Bromkomplex und/oder eine Brom abgebende Verbindung.

2. Zusammensetzung nach Anspruch 1, **dadurch gekennzeichnet, dass** sie des Weiteren mindestens einen Polyglycolether, optional einen Poly(Ethylenglycol)-Ether oder einen Poly(Propylenglycol)-Ether umfasst.

3. Zusammensetzung nach Anspruch 1 oder Anspruch 2, **dadurch gekennzeichnet, dass** die oder jede langkettige Alkyltriaminverbindung eine Verbindung umfasst, die folgende allgemeine Formel aufweist: R-N((CH₂)ₘNH₂)₂.

4. Zusammensetzung nach einem der vorhergehenden Ansprüche, **dadurch gekennzeichnet, dass** m und n gleich 3 ist.

5. Zusammensetzung nach einem der vorhergehenden Ansprüche, **dadurch gekennzeichnet, dass** die oder jede langkettige Alkyltriaminverbindung eine lineare oder verzweigte Alkylkette aufweist, die zehn bis sechzehn Kohlenstoffatome umfasst.

6. Zusammensetzung nach einem der vorhergehenden Ansprüche, **dadurch gekennzeichnet, dass** der eine oder die mehreren aliphatischen Alkohole jeweils ein bis vier Kohlenstoffatome umfassen.

7. Zusammensetzung nach einem der vorhergehenden Ansprüche, **dadurch gekennzeichnet, dass** der oder jeder des einen oder der mehreren aliphatischen Alkohole ein Primäralkohol ist.

8. Zusammensetzung nach einem der vorhergehenden Ansprüche, **dadurch gekennzeichnet, dass** sie zwei verschiedene aliphatische Alkohole, optional Ethanol und *n*-Propanol umfasst.

9. Zusammensetzung nach einem der vorhergehenden Ansprüche, **dadurch gekennzeichnet, dass** sie 5 Vol.-% bis 25 Vol.-% aliphatische Alkohole, optional 10 Vol.-% bis 20 Vol.-% aliphatische Alkohole umfasst.

10. Zusammensetzung nach einem der vorhergehenden Ansprüche, **dadurch gekennzeichnet, dass** sie bis zu 0,1 Gew.-% Brom umfasst.

11. Zusammensetzung nach einem der vorhergehenden Ansprüche, **dadurch gekennzeichnet, dass** sie 0,01 Gew.-% bis 0.05 Gew.-% Brom umfasst.

12. Zusammensetzung nach einem der vorhergehenden Ansprüche, **dadurch gekennzeichnet, dass** sie 10 Vol.-% bis 30 Vol.-% der langkettigen Alkyltriaminverbindung oder -verbindungen, optional 15 Vol.-% bis 25 Vol.-% davon umfasst.

13. Zusammensetzung zur Reinigung von Textilien und Dergleichen, **dadurch gekennzeichnet, dass** sie mindestens eine langkettige Alkyltriaminverbindung umfasst, die folgende allgemeine Formel aufweist: R-N((CH₂)ₘNH₂)(CH₂)ₙNH₂, wobei R eine lineare oder verzweigte Alkylkette ist, die Folgendes umfasst: mindestens acht Kohlenstoffatome, wobei m und n gleich 2, 3 oder 4 ist, ein oder mehrere aliphatische Alkohole und Brom, einen Bromkomplex und/oder eine Brom abgebende Verbindung.

14. Geschirrspülzusammensetzung, **dadurch gekennzeichnet, dass** sie mindestens eine langkettige Alkyltriaminverbindung umfasst, die folgende allgemeine Formel aufweist: R-N((CH₂)ₘNH₂)(CH₂)ₙNH₂, wobei R eine lineare oder verzweigte Alkylkette ist, die Folgendes umfasst: mindestens acht Kohlenstoffatome, wobei m und n gleich 2, 3 oder 4 ist, ein oder mehrere aliphatische Alkohole und Brom, einen Bromkomplex und/oder eine Brom abgebende Verbindung.

15. Oberflächenbeschichtungszusammensetzung, **dadurch gekennzeichnet, dass** sie mindestens eine langkettige Alkyltriaminverbindung umfasst, die folgende allgemeine Formel aufweist: R-N((CH₂)ₘNH₂)(CH₂)ₙNH₂, wobei R eine lineare oder verzweigte Alkylkette ist, die Folgendes umfasst: mindestens acht Kohlenstoffatome, wobei m und n gleich 2, 3 oder 4 ist, ein oder mehrere aliphatische Alkohole und Brom, einen Bromkomplex und/oder eine Brom abgebende Verbindung.

## Revendications

1. Composition aqueuse nettoyante et désinfectante de surface, **caractérisée en ce qu'**elle comprend au moins un composé de polyamine alkyle à chaîne longue présentant la formule générale R-N((CH₂)ₘNH₂)(CH₂)ₙNH₂, dans laquelle R est une chaîne alkyle linéaire ou ramifiée comprenant au moins huit atomes de carbone, et m et n sont chacun égaux à 2, 3 ou 4, un ou plusieurs alcools aliphatiques, et du brome, un complexe de brome et/ou un composé libérant du brome.

2. Composition selon la revendication 1, **caractérisée en ce qu'**elle comprend en outre au moins un éther de polyglycol, éventuellement un éther de poly(éthylène glycol) ou un éther de poly(propylène glycol).

3. Composition selon la revendication 1 ou 2, **caractérisée en ce que** le ou chaque composé de triamine alkyle à chaîne longue comprend un composé présentant la formule générale R-N((CH₂)ₘNH₂)₂.

4. Composition selon l'une quelconque des revendications précédentes, **caractérisée en ce que** m et n sont chacun égaux à 3.

5. Composition selon l'une quelconque des revendications précédentes, **caractérisée en ce que** le ou chaque composé de triamine alkyle à chaîne longue présente une chaîne alkyle linéaire ou ramifiée comprenant entre dix et seize atomes de carbone.

6. Composition selon l'une quelconque des revendications précédentes, **caractérisée en ce que** lesdits un ou plusieurs alcools aliphatiques comprennent chacun entre un et quatre atomes de carbone.

7. Composition selon l'une quelconque des revendications précédentes, **caractérisée en ce que** le ou chacun desdits un ou plusieurs alcools aliphatiques est un alcool primaire.

8. Composition selon l'une quelconque des revendications précédentes, **caractérisée en ce qu'**elle comprend deux alcools aliphatiques différents, éventuellement l'éthanol et le *n*-propanol.

9. Composition selon l'une quelconque des revendications précédentes, **caractérisée en ce qu'**elle comprend entre 5 et 25 % en volume d'alcools aliphatiques, éventuellement entre 10 et 20 % d'alcools aliphatiques.

10. Composition selon l'une quelconque des revendications précédentes, **caractérisée en ce qu'**elle comprend jusqu'à 0,1 % en poids de brome.

11. Composition selon l'une quelconque des revendications précédentes, **caractérisée en ce qu'**elle comprend entre 0,01 et 0,05 % en poids de brome.

12. Composition selon l'une quelconque des revendications précédentes, **caractérisée en ce qu'**elle comprend entre 10 et 30 % en volume de composé(s) de triamine alkyle à chaîne longue, éventuellement entre 15 et 25 % en volume.

13. Composition permettant de nettoyer des textiles et similaires, **caractérisée en ce qu'**elle comprend au moins un composé de triamine alkyle à chaîne longue présentant la formule générale R-N((CH₂)ₘNH₂)(CH₂)ₙNH₂, dans laquelle R est une chaîne alkyle linéaire ou ramifiée comprenant au moins huit atomes de carbone, et m et n sont chacun égaux à 2, 3 ou 4, un ou plusieurs alcools aliphatiques, et du brome, un complexe de brome et/ou un composé libérant du brome.

14. Composition de nettoyage de vaisselle, **caractérisée en ce qu'**elle comprend au moins un composé de triamine alkyle à chaîne longue présentant la formule générale R-N((CH₂)ₘNH₂)(CH₂)ₙNH₂, dans laquelle R est une chaîne alkyle linéaire ou ramifiée comprenant au moins huit atomes de carbone, et m et n sont chacun égaux à 2, 3 ou 4, un ou plusieurs alcools aliphatiques, et du brome, un complexe de brome et/ou un composé libérant du brome.

15. Composition de revêtement de surface, **caractérisée en ce qu'**elle comprend au moins un composé de triamine alkyle à chaîne longue présentant la formule générale R-N((CH₂)ₘNH₂)(CH₂)ₙNH₂, dans laquelle R est une chaîne alkyle linéaire ou ramifiée comprenant au moins huit atomes de carbone, et m et n sont chacun égaux à 2, 3 ou 4, un ou plusieurs alcools aliphatiques, et du brome, un complexe de brome et/ou un composé libérant du brome.
